Europäisches Patentamt

European Patent Office

Office européen des brevets

(19)

(11) Numéro de publication: **0 095 395**
**B1**

(12) **FASCICULE DE BREVET EUROPEEN**

(45) Date de publication du fascicule du brevet:
**23.10.85**

(51) Int. Cl.⁴: **B 01 D 53/00,** C 07 C 7/163,
C 07 C 9/04

(21) Numéro de dépôt: **83400856.7**

(22) Date de dépôt: **28.04.83**

(54) Procédé d'hydrodésulfuration et de désoxygénation d'un gaz contenant de l'oxygène et des composés organiques du soufre.

(30) Priorité: **25.05.82 FR 8209210**

(43) Date de publication de la demande:
**30.11.83 Bulletin 83/48**

(45) Mention de la délivrance du brevet:
**23.10.85 Bulletin 85/43**

(84) Etats contractants désignés:
**BE DE GB IT NL**

(56) Documents cités:
**DE - A - 2 731 611**
**US - A - 4 260 840**

**CHEMICAL ABSTRACTS, vol. 69, no. 2, 1968, page 410, no. 4179n, Columbus, Ohio, US**

(73) Titulaire: **INSTITUT FRANCAIS DU PETROLE, 4, Avenue de Bois-Préau, F-92502 Rueil-Malmaison (FR)**

(72) Inventeur: **Deschamps, André, 3, Chemin des Princes, F-78590 Noisy le Roi (FR)**
Inventeur: **Cosyns, Jean, 50, route d'Herbeville, F-78580 Maule (FR)**
Inventeur: **Le Page, Jean-François, 13, rue des Primevères, F-92500 Rueil Malmaison (FR)**
Inventeur: **Hotier, Gérard, 18, boulevard du Maréchal Foch, F-92500 Rueil Malmaison (FR)**

BUNDESDRUCKEREI BERLIN

## Description

L'invention concerne un procédé permettant d'effectuer l'hydrodésulfuration et la désoxygénation d'un gaz, renfermant simultanément de l'oxygène et des composés organiques du soufre.

Le gaz naturel, distribué par réseau, renferme des traces de composés organiques du soufre, provenant d'une désulfuration incomplète ou introduits volontairement pour donner une odeur au gaz (généralement du tétrahydrothiophène (THT) ou des mercaptans). Malgré leur faible concentration dans le gaz (par exemple 10 à 30 mg de THT par normal m$^3$ de gaz), des composés soufrés sont gênants lorsque le gaz est utilisé en présence de catalyseurs sensibles au soufre. C'est notamment le cas lorsque ce gaz est utilisé pour fabriquer de l'hydrogène ou du gaz de synthèse par reformage à la vapeur d'eau, par exemple dans les usines de fabrication d'ammoniac ou de méthanol.

Généralement ce gaz est alors désulfuré par traitement à l'hydrogène en présence d'un catalyseur d'hydrodésulfuration assurant la transformation du ou des composés organiques du soufre en hydrogène sulfuré qui peut ensuite être facilement séparé ou capté par une masse à base d'oxyde de zinc, par exemple. Les catalyseurs utilisés dans l'étape d'hydrodésulfuration sont généralement à base de cobalt et molybdène ou nickel et molybdène sur un support d'alumine.

Le gaz de réseau peut parfois contenir, en plus, de faibles quantités d'oxygène provenant d'additions d'air effectuées pour ajuster le pouvoir combustible des gaz trop riches. La teneur en oxygène est généralement comprise entre 0,05 et 1% en volume. On observe dans ces conditions un mauvais fonctionnement des catalyseurs d'hydrodésulfuration: une partie du soufre est transformé en soufre élémentaire et/ou en dioxyde de soufre qui ne sont pas captés par la masse d'oxyde de zinc et empoisonnent les catalyseurs utilisés dans le traitement ultérieur du gaz.

D'autres gaz qui peuvent renfermer à la fois de l'oxygène et des composés organiques du soufre (par exemple COS, CS$_2$, mercaptans, sulfures, disulfures) sont, par exemple, les gaz provenant de la cokéfaction et de la gazéification de charbon, lignite, schistes bitumineux ou résidus pétroliers, ainsi que les gaz associés des puits de pétrole ou les gaz de raffinerie.

Pour remédier à cet inconvénient, deux solutions ont déjà été proposées.

Le brevet US-4 181 503 propose d'éliminer dans une première étape l'oxygène par traitement du gaz additionné d'hydrogène sur un catalyseur à base de platine, puis dans une deuxième étape de traiter le gaz désoxygéné sur un catalyseur à base de nickel et molybdène pour transformer le soufre organique en hydrogène sulfuré. Ce procédé a l'inconvénient d'être coûteux car, d'une part, il emploie un catalyseur au platine et, d'autre part, le passage successif sur les deux catalyseurs ne peut pas toujours se faire à l'intérieur d'un réacteur déjà existant conçu seulement pour l'hydrodésulfuration de gaz exempt d'oxygène.

La demande de brevet français FR-A-2 521 448 (publiée le 19. 8. 1983) propose de réaliser simultanément ces deux opérations par traitement du gaz additionné d'hydrogène (ou plus avantageusement d'un gaz industriel renfermant à la fois de l'hydrogène et du monoxyde de carbone) sur un catalyseur à base de palladium.

On a maintenant découvert, et c'est ce qui fait l'objet de l'invention, que l'association d'un catalyseur à base de palladium et d'un catalyseur classique d'hydrodésulfuration à base de molybdène et de nickel et/ou cobalt permet de réaliser, dans des conditions particulièrement avantageuses, la désoxygénation et l'hydrodésulfuration.

L'invention concerne, plus particulièrement, un procédé d'hydrodésulfuration et de désoxygénation d'un gaz (A) renfermant à la fois, du méthane, de l'oxygène et au moins un composé organique du soufre, caractérisé en ce que l'on fait passer un mélange dudit gaz et d'un gaz (B) renfermant de l'hydrogène sur un premier catalyseur de palladium, à une température de 300° à 450°C, puis sur un second catalyseur renfermant du molybdène et au moins un second métal du groupe formé par le nickel et le cobalt, à une température comprise entre les limites précitées de 300° à 450°C.

Selon une forme particulière de mise en oeuvre de l'invention, un gaz contenant plus de 2 mg/Nm$^3$, par exemple, de 2 à 20 mg/Nm$^3$, particulièrement de 4 à 12 mg/Nm$^3$ de soufre sous forme de composés organiques du soufre et de 0,05 à 2% en volume, plus particulièrement de 0,1 à 1% en volume d'oxygène est traité par un gaz renfermant de l'hydrogène, à une température de 300° à 450°C, de préférence 320° à 400°C, d'abord par un catalyseur de palladium puis par un catalyseur de cobalt-molybdène et/ou nickel-molybdène.

Le premier catalyseur (palladium) représente, en volume, de 20 à 60%, de préférence 30 à 50%, du volume total des catalyseurs, le second catalyseur représentant respectivement 80 à 40%, de préférence 70 à 50% de ce volume total.

De préférence, les deux catalyseurs sont disposés dans le même réacteur, ce qui représente une économie considérable en investissement et coût de fonctionnement.

Le gaz d'hydrogénation peut être de l'hydrogène relativement pur ou, plus avantageusement, un gaz industriel renfermant à la fois de l'hydrogène et du monoxyde de carbone.

Le premier catalyseur est constitué de 0,1 à 5% en poids de palladium déposé sur (ou incorporé à) tout support convenable tel que, par exemple, l'alumine, la silice ou une silice-alumine, la surface spécifique de ce support (méthode BET) étant comprise de préférence entre 5 et 400 m$^2$/g. On peut utiliser toute méthode appropriée pour incorporer le palladium telle que notamment: par imprégnation

2

avec une solution aqueuse d'un composé de palladium, par exemple une solution aqueuse de nitrate, de chlorure, ou de complexe aminé du type Pd(NH$_3$)$_4$X$_2$ (X étant un anion) ou bien encore avec une solution constituée par un organo complexe tel que l'acétylacétonate de palladium dissous dans un composé organique approprié.

Après l'imprégnation, le composé de palladium est traité de toute manière convenable pour obtenir le métal réduit, par exemple par calcination à l'air suivie de réduction sous hydrogène.

On peut aussi, préalablement à son emploi, traiter le catalyseur avec un mélange d'hydrogène et de composé sulfuré tel que l'H$_2$S par exemple.

Les catalyseurs de nickel-molybdène ou cobalt-molybdène sont bien connus et ne nécessitent pas de description détaillée. Ils renferment habituellement 5 à 30% en poids de molybdène, calculé en MoO$_3$, et 1 à 10% en poids de nickel et/ou cobalt, calculé en NiO ou CoO, le complément étant un support, par exemple une alumine, une silice ou une silice-alumine de surface comprise entre 50 et 400 m$^2$/g. On préfère l'alumine.

La vitesse spatiale, c'est-à-dire le volume de gaz (compté dans les conditions normales de température et de pression) traité par volume de catalyseur et par heure est comprise entre 1000 et 25 000 et plus généralement entre 4000 et 16 000 pour chacun des deux catalyseurs pris isolément.

La quantité d'hydrogène est au moins égale à la quantité théoriquement nécessaire à la conversion des composés organiques du soufre en hydrogène sulfuré et à la transformation de l'oxygène en vapeur d'eau. Elle est de préférence au moins égale à deux fois cette quantité, et généralement de 2 à 6% en volume de la quantité de gaz naturel.

La pression opératoire peut varier largement, par exemple entre 1 et 80 bars. On préfère généralement opérer à la pression du réseau ou à celle du procédé utilisant le gaz, c'est-à-dire généralement entre 20 et 60 bars.

Les composés organiques du soufre qui peuvent être éliminés par le procédé de l'invention sont, par exemple:

— le tétrahydrothiophène,
— les mercaptans de formule R—SH où R = hydrocarbyl de 1—6 atomes de carbone, par exemple le methylmercaptan, l'éthylmercaptan, les propylmercaptans et les butylmercaptans,
— les sulfures de formule R$_1$ R$_2$ S où R$_1$ et R$_2$ = chacun hydrocarbyl de 1—4 atomes de carbone, par exemple le sulfure de méthyle, le sulfure d'éthyle et le sulfure de butyle,
— les disulfures de formule R$_1$ S S R$_2$ où R$_1$ et R$_2$ = chacun hydrocarbyl de 1—4 atomes de carbone, par exemple le diméthyldisulfure, le diéthyldisulfure et le dipropyldisulfure.

L'exemple suivant, non limitatif, est donné pour illustrer l'invention.


Exemple


On a comparé le fonctionnement d'un catalyseur du commerce à base de nickel-molybdène (14% de MoO$_3$ et 3% de NiO, en poids) sur alumine et d'un catalyseur du commerce à base de cobalt-molybdène (14% MoO$_3$ et 3% CoO en poids) sur alumine à celui d'un catalyseur à base de palladium (0,3% Pd en poids) sur alumine employé soit seul, soit suivi d'un catalyseur du commerce à base de nickel-molybdène (14% de MoO$_3$ et 3% NiO, en poids) sur alumine de surface égale à 150 m$^2$/g, ou d'un catalyseur du commerce à base de cobalt molybdène (14% de MoO$_3$ et 3% CoO, en poids).

Le catalyseur au palladium est préparé de la manière suivante. On imprègne un support d'alumine de 70 m$^2$/g de surface spécifique et de 0,6 cm$^3$ par gramme de volume poreux avec une solution de nitrate de palladium, de manière à obtenir sur le catalyseur fini 0,3% en poids de palladium métal. Après imprégnation, le catalyseur est séché puis calciné à 450°C pendant deux heures sous courant d'air. Avant utilisation, le catalyseur est réduit à 200°C par un courant d'hydrogène pendant deux heures.

Le gaz traité est un gaz naturel essentiellement constitué de méthane et renfermant 25 mg/Nm$^3$ de tétrahydrothiophène (THT) et 0,5% en volume d'oxygène.

Dans un réacteur tubulaire en acier de 3 cm de diamètre intérieur, chauffé électriquement, on place:

— Soit 100 cm$^3$ de catalyseur CoMo
— Soit 100 cm$^3$ de catalyseur NiMo
— Soit 100 cm$^3$ de catalyseur à base de palladium
— Soit 50 cm$^3$ de catalyseur à base de palladium
suivi de 50 cm$^3$ de catalyseur NiMo ou de 50 cm$^3$ de catalyseur CoMo.

Au sommet du réacteur, on injecte 500 N l/h du gaz naturel et 15 N l/h d'hydrogène sous une pression de 30 bars.

Le gaz sortant au bas du réacteur est détendu et analysé pour déterminer ses teneurs en THT, H$_2$S, soufre élémentaire, SO$_2$, soufre organique et oxygène.

## 0 095 395

Le tableau suivant donne les résultats obtenus avec chacun des catalyseurs, pour différentes températures, après une période de mise en régime de 100 heures.

On constate que le catalyseur au palladium employé soit seul, soit en association avec les catalyseurs NiMo ou CoMo assure une conversion pratiquement complète du THT dans l'intervalle de température de 300°C à 400°C, plus particulièrement de 350°C à 400°C. Dans le cas du catalyseur au palladium employé seul on note cependant des traces de soufre organique qui peuvent être gênantes, par contre avec l'association des catalyseurs au palladium et au NiMo ou CoMo, ces traces de produits organosoufrés disparaissent.

Au contraire le catalyseur à base de cobalt-molybdène ou à base de nickel-molybdène fournit à toute température un gaz contenant, en plus de traces d'hydrogène sulfuré, du dioxyde de soufre et du soufre élémentaire. Ceci semble en relation avec le fait que le gaz sortant renferme toujours de l'oxygène. De plus, à chaque augmentation de température, on constate une augmentation passagère de la teneur en dioxyde de soufre due probablement à l'oxydation des sulfures de cobalt (ou nickel) et de molybdène présents sur le catalyseur.

De plus on a vérifié que la présence de $CO_2$ dans le gaz à traiter n'entraînait pas de réaction de méthanation et que les hydrocarbures supérieurs tels que l'éthane, le propane et les butanes ne subissaient pas d'hydrogénolyse. On ne risque donc pas d'avoir des consommations d'hydrogène excessives du fait de ces réactions.

| Catalyseur | T | THT | $H_2S$ | Soufre élémentaire | $SO_2$ | Soufre organique total | $O_2$ |
|---|---|---|---|---|---|---|---|
| | °C | mg/Nm$^3$ | mg/Nm$^3$ | mg/Nm$^3$ | mg/Nm$^3$ | mg/Nm$^3$ | ppm vol. |
| $Pd/Al_2O_3$ | 300 | <0,05 | 9 | 0,1 | 1 | 0,4 | 15 |
| | 350 | <0,05 | 9,35 | nd | nd | 0,3 | nd |
| | 400 | <0,05 | 9,53 | nd | nd | 0,1 | nd |
| 50% $Pd/Al_2O_3$ | 300 | <0,05 | 9 | nd | nd | 0,2 | 15 |
| 50% $NiMo/Al_2O_3$ | 350 | <0,05 | 9,60 | nd | nd | <0,1 | nd |
| | 400 | <0,05 | 9,62 | nd | nd | <0,1 | nd |
| $NiMo/Al_2O_3$ | 300 | 6 | 4 | 2 | 8 | 6 | 4300 |
| | 350 | <0,05 | 2 | 1 | 12 | 2 | 4000 |
| | 400 | <0,05 | 1 | 0,5 | 14 | 2,7 | 700 |
| 50% $Pd/Al_2O_3$ | 300 | <0,05 | 9,1 | nd | nd | 0,25 | 20 |
| 50% $CoMo/Al_2O_3$ | 350 | <0,05 | 9,55 | nd | nd | <0,1 | 10 |
| | 400 | <0,05 | 9,65 | nd | nd | <0,1 | nd |
| $CoMo/Al_2O_3$ | 300 | 5 | 3 | 1,5 | 6,5 | 9 | 4500 |
| | 350 | <0,05 | 1 | 0,5 | 14,1 | 4,4 | 4100 |
| | 400 | <0,05 | 2 | 1 | 13 | 3,7 | 1000 |

nd: non détecté

## Revendications

1. Procédé d'hydrodésulfuration et de désoxygénation d'un gaz (A) renfermant à la fois, du méthane, de l'oxygène et au moins un composé organique du soufre, caractérisé en ce que l'on fait passer un mélange dudit gaz et d'un gaz (B) renfermant de l'hydrogène sur un premier catalyseur de palladium, à une température de 300° à 450°C, puis sur un second catalyseur renfermant du molybdène et au moins un second métal du groupe formé par le nickel et le cobalt, à une température comprise entre les limites précitées de 300° à 450°C.

2. Procédé selon la revendication 1, dans lequel le gaz (A) renferme au moins 2 mg/Nm$^3$ de soufre sous forme de composé organique du soufre et de 0,05 à 2% en volume d'oxygène.

3. Procédé selon la revendication 2, dans lequel le gaz (A) renferme de 2 à 20 mg/Nm$^3$ de soufre sous forme de composé organique du soufre.

4. Procédé selon l'une des revendications 1 à 3, dans lequel la temperature est de 320 à 400°C.

5. Procédé selon l'une des revendications 1 à 4, dans lequel la quantité d'hydrogène est au moins égale à la quantité théoriquement nécessaire à la conversion du composé organique du soufre en hydrogène sulfuré et à la conversion de l'oxygène en vapeur d'eau.

4

6. Procédé selon l'une des revendications 1 à 4, dans lequel la quantité d'hydrogène représente 2 à 6% du volume de gaz (A).

7. Procédé selon l'une des revendications 1 à 6, dans lequel le premier catalyseur renferme de 0,1 à 5% en poids de palladium sur alumine ou silice et le second catalyseur de 5 à 30% en poids de molybdène, calculé en $MoO_3$, et de 1 à 10% en poids de nickel et/ou cobalt, calculé en NiO et/ou CoO, sur alumine, silice ou silice-alumine.

8. Procédé selon l'une des revendications 1 à 7, dans lequel le premier catalyseur et le second catalyseur représentent respectivement 20 à 60% et 80 à 40% du volume total des catalyseurs.

9. Procédé selon l'une des revendications 1 à 8, dans lequel le débit horaire de gaz traité est de 1000 à 25 000 volumes (compté dans les conditions normales de température et de pression) par volume de catalyseur pour chacun des deux catalyseurs pris isolément.

10. Procédé selon l'une des revendications 1 à 9, dans lequel le gaz A renferme au moins 2 mg/$Nm^3$ de soufre sous forme de tétrahydrothiophène et de 0,1 à 1% en volume d'oxygène.

## Patentansprüche

1. Verfahren zur Hydroentfernung von Schwefel und Sauerstoff aus einem gleichzeitig Methan, Sauerstoff und wenigstens eine organische Verbindung des Schwefels enthaltenden Gas (A), dadurch gekennzeichnet, daß man ein Gemisch dieses Gases und eines Wasserstoff enthaltenden Gases (B) über einen ersten Palladiumkatalysator bei einer Temperatur von 300 bis 450°C, dann über einen zweiten, Molybdän und wenigstens ein zweites Metall der durch Nickel und Kobalt gebildeten Gruppe enthaltenden Katalysator bei einer Temperatur zwischen den genannten Grenzwerten von 300 und 450°C strömen läßt.

2. Verfahren nach Anspruch 1, dadurch gekennzeichnet, daß das Gas (A) wenigstens 2 mg/$Nm^3$ Schwefel in Form einer organischen Verbindung des Schwefels und 0,05 bis 2 Volumen-% Sauerstoff enthält.

3. Verfahren nach Anspruch 2, dadurch gekennzeichnet, daß das Gas (A) 2 bis 20 mg/$Nm^3$ Schwefel in Form einer organischen Schwefelverbindung enthält.

4. Verfahren nach einem der Ansprüche 1 bis 3, dadurch gekennzeichnet, daß die Temperatur bei 320 bis 400°C liegt.

5. Verfahren nach einem der Ansprüche 1 bis 4, dadurch gekennzeichnet, daß die Wasserstoffmenge wenigstens gleich der theoretisch zur Umwandlung der organischen Verbindung des Schwefels in Schwefelwasserstoff und zur Umwandlung des Sauerstoffs in Wasserdampf notwendigen Menge ist.

6. Verfahren nach einem der Ansprüche 1 bis 4, dadurch gekennzeichnet, daß die Wasserstoffmenge 2 bis 6 Volumen-% des Gases (A) ausmacht.

7. Verfahren nach einem der Ansprüche 1 bis 6, dadurch gekennzeichnet, daß der erste Katalysator 0,1 bis 5 Gew.-% Palladium auf Aluminiumoxid oder Siliziumdioxid und der zweite Katalysator 5 bis 30 Gew.-% Molybdän, berechnet als $MoO_3$ und 1 bis 10 Gew.-% Nickel und/oder Kobalt, berechnet als NiO und/oder CoO auf Aluminiumoxid, Siliziumdioxid oder Siliziumdioxid-Aluminiumoxid enthält.

8. Verfahren nach einem der Ansprüche 1 bis 7, dadurch gekennzeichnet, daß der erste Katalysator und der zweite Katalysator jeweils 20 bis 60% und 80 bis 40% des Gesamtvolumens der Katalysatoren darstellen.

9. Verfahren nach einem der Ansprüche 1 bis 8, dadurch gekennzeichnet, daß die stündliche Geschwindigkeit des Gases 1000 bis 25 000 Vol. (unter normalen Bedingungen der Temperatur und des Druckes) pro Vol. des Katalysators für jeden Katalysator.

10. Verfahren nach einem der Ansprüche 1 bis 9, dadurch gekennzeichnet, daß das Gas A wenigstens 2 mg/$Nm^3$ Schwefel wie Tetrahydrothiophen und 0,1 bis 1 Vol.-% Sauerstoff enthält.

## Claims

1. A process for hydrodesulfurizing and deoxygenating a gas (A) comprising together methane, oxygen and at least one organic sulfur compound, characterized in passing a mixture of said gas with a hydrogen-containing gas (B) on a first palladium catalyst, at a temperature of 300 to 450°C, then on a second catalyst comprising molybdenum and at least one second metal of the group consisting of nickel and cobalt, at a temperature comprised between the above limits of 300 and 450°C.

2. A process according to claim 1, wherein the gas (A) comprises at least 2 mg of sulfur, as organic sulfur compound, per $Nm^3$ and 0.05 to 2% by volume of oxygen.

3. A process according to claim 2, wherein the gas (A) comprises 2 to 20 mg of sulfur, as organic sulfur compound, per $Nm^3$.

4. A process according to one of claims 1 to 3, wherein the temperature is from 320 to 400°C.

5. A process according to one of claims 1 to 4, wherein the hydrogen proportion is at least the theoretical proportion necessary to the conversion of the organic sulfur compound to hydrogen sulfide and to the conversion of oxygen to steam.

6. A process according to one of claims 1 to 4, wherein the hydrogen amount represents 2 to 6% by volume of the gas (A).

7. A process according to one of claims 1 to 6, wherein the first catalyst comprises 0.1 to 5% b.w. of palladium on alumina or silica and the second catalyst 5 to 30% b.w. of molybdenum, calculated as $MoO_3$, and 1 to 10% b.w. of nickel and/or cobalt, calculated as NiO and/or CoO, on alumina, silica or silica-alumina.

8. A process according to one of claims 1 to 7, wherein the first catalyst and the second catalyst represent respectively 20 to 60% and 80 to 40% of the total volume of the catalysts.

9. A process according to one of claims 1 to 8, wherein the hourly feed rate of the treated gas in from 1000 to 25 000 volumes (in the normal conditions of temperature and pressure) per volume of catalyst for each of the two catalysts considered separately.

10. A process according to one of claims 1 to 9, wherein the gas A comprises at least 2 mg/$Nm^3$ of sulfur as tetrahydrothiophene and from 0.1 to 1% by volume of oxygen.